# EUROPEAN PATENT SPECIFICATION

(11) **EP 4 260 865 B1**
(45) Date of publication and mention of the grant of the patent: **10.09.2025**
(21) Application number: 23167248.6
(22) Date of filing: 11.04.2023
(51) Int. Cl.: A61K 36/718, A61K 36/29, A61K 36/39, A61K 36/63, A23L 33/105, A61P 3/06

(54) **COMPOSITION COMPRISING PLANT EXTRACTS FOR TREATING HYPERCHOLESTEROLEMIA**
ZUSAMMENSETZUNG AUS PFLANZENEXTRAKTEN ZUR BEHANDLUNG VON HYPERCHOLESTERINÄMIE
COMPOSITION COMPRENANT DES EXTRAITS VEGETAUX POUR LE TRAITEMENT DE L'HYPERCHOLESTEROLEMIE

(30) Priority: 13.04.2022 IT 202200007388
(43) Date of publication of application: 18.10.2023
(73) Proprietor: Anvest Group S.r.l., 20152 Milano (IT)
(72) Inventor: ALFIERI, Sandro, 84083 Castel San Giorgio (IT); ALFIERI, Dario, 84086 Roccapiemonte (IT); ALFIERI, Roberto, 84086 Roccapiemonte (IT)
(74) Representative: Cattaneo, Elisabetta

(56) References cited:
- EP-A1- 3 590 356
- WO-A1-2015/136441
- WO-A2-2021/090214
- MBOUCHE FANMOE MARCÉLINE JOËLLE ET AL: "Ipomea batatas Leaf Powder from Cameroon: Antioxidant Activity and Antihyperlipidemic Effect in Rats Fed with a High-Fat Diet", JOURNAL OF LIPIDS, vol. 2021, 10 June 2021 (2021-06-10), pages 1 - 11, XP055977974, ISSN: 2090-3030, DOI: 10.1155/2021/5539878
- LOCKYER STACEY ET AL: "Impact of phenolic-rich olive leaf extract on blood pressure, plasma lipids and inflammatory markers: a randomised controlled trial", EUROPEAN JOURNAL OF NUTRITION, STEINKOPFF VERLAG, DARMSTADT, DE, vol. 56, no. 4, 7 March 2016 (2016-03-07), pages 1421 - 1432, XP036256931, ISSN: 1436-6207, [retrieved on 20160307], DOI: 10.1007/S00394-016-1188-Y

## Description

### FIELD OF THE INVENTION

The present invention relates to a composition comprising natural ingredients for the treatment of hypercholesterolemia.

### STATE OF THE ART

Cholesterol is a molecule of the sterol class, synthesized by the liver and absorbed from the intestine with the diet. Cholesterolemia is the concentration of cholesterol in the blood and is an index of the correct synthesis and use of the molecule by the body.

Cholesterol functions are closely related to the various phases of cellular physiology. In fact, cholesterol:
- As an essential element of the cell membrane, is inserted in the phospholipid bilayer with the -OH groups oriented towards the phospholipid polar heads, thus reducing and regulating the fluidity thereof, increasing the mechanical stability of the cell and decreasing the permeability to small water-soluble molecules;
- aggregates with some membrane proteins forming transport vesicles;
- is the precursor for many steroid molecules such as some hormones like aldosterone, cortisone, testosterone, estradiol;
- is also a component of bile, essential for the digestion of fats.

All cells in the body are capable of synthesizing cholesterol from acetyl-Coenzyme A. However, most of the molecule is produced by liver cells and transported through the bloodstream to peripheral districts. The chemical reactions leading to the synthesis of cholesterol follow the mevalonate pathway.

In the synthesis of cholesterol, the first step is represented by the conversion of acetyl-CoA into mevalonate. A series of intermediate chemical reactions take place in which two molecules of Acetyl-CoA initially combine to form aceto-acetyl-CoA. The double molecule reacts with another acetyl-CoA molecule, generating 3-hydroxy-3-methylglutaryl-CoA (HMG-CoA). This is the reaction intermediate for the synthesis of cholesterol through the action of the enzyme hydroxymethyl-CoA reductase.

The fatty nature of cholesterol makes it impossible for it to be dissolved in the blood, which is mostly made of water. The need therefore arises to transport cholesterol within lipoproteins designed for the transport of lipids. They are synthesized in the intestinal epithelium in the prandial phase in the form of chylomicrons, and in the liver during fasting in the form of VLDL.

Lipoproteins designed for the transport of lipids, and in this case of cholesterol, are classified according to their composition and the lipid/protein ratio present therein. Lipoproteins whose structure is mostly made of lipids will have a low density (LD), lipoproteins mainly made of proteins will instead have a high density (HD).

Based on this concept, lipoproteins are divided into 4 groups:
- Chylomicrons: low-density lipoproteins produced by the intestine which represent the main carrier of lipids from the alimentary canal to the liver.
- VLDL: lipoproteins synthesized in the liver, the acronym stands for Very Low-Density Lipoprotein, they are mainly made of triglycerides. When their density decreases, they generate LDL lipoproteins.
- LDL: lipoproteins deriving from VLDL due to the loss of a lipid portion. They mainly transport cholesterol to the cells, where they find specific receptors.
- HDL: they are high protein content (high density) lipoproteins responsible for the reverse transport of cholesterol to the liver.

Blood cholesterol is the sum of cholesterol from endogenous synthesis (in the liver) and dietary absorption (in the intestine). By inhibiting the two ways, the result is the control of the optimal levels of circulating cholesterol and consequent reduction of the cardio- and cerebrovascular risks. High levels of LDL cholesterol in the blood stream lead to cardiovascular risk exposure. LDL cholesterol migrates from the blood stream to the tunica intima of the vessel wall, where it is exposed to oxidation by free radicals. This process gives rise to inflammatory processes (pro-inflammatory cytokine synthesis) which attract macrophages. These immune molecules migrate into the tunica intima and begin the oxidized LDL phagocytosis, generating foam cells which narrow the vessel lumen, thus occluding the blood circulation and causing stenosis (lipid streak). This leads to the formation of atheromatous plaques. The risk associated with the presence of plaques in the endothelium of blood vessels lies in the fact that they thin the vessel lumen, thus reducing the volume of blood in transit, and with it the amount of oxygen and useful substances transported in the affected areas.

We speak of hypercholesterolemia when total cholesterol (LDL + HDL) exceeds safe limits. Desirable values are as follows:
- total cholesterol: up to 200 mg/dL
- LDL cholesterol: up to 130 mg/dL
- HDL cholesterol: not less than 50 mg/dL
- Triglycerides: up to 150 mg/dL

The risk factors that predispose to hypercholesterolemia range from diet (intake of considerable amounts of foods high in cholesterol), overweight and obesity, lack of physical activity (a dynamic and sporty lifestyle increases the HDL-good cholesterol levels), diabetes, metabolic diseases, hereditary factors. Particular attention should be paid to the hereditary nature of the disease, since most of the cholesterol in the circulation has an endogenous origin, i.e. it is synthesized by our body. A high hepatic production of the molecule, or its poor elimination from the blood stream due to the limited functioning of the hepatic receptors for cholesterol, can play a key role in its accumulation inside the vessels. Every year in Europe there are 4 million deaths from cardiovascular diseases affecting women in 55% of cases. Considering the European Union alone, deaths from these pathologies amount to 1.8 million a year and the expenditure relating to cardiovascular diseases stands at 210 billion euros, 53% of which is generated by clinical management. Intervention studies have shown that if it were possible to eliminate all risk factors, cardiovascular events could be reduced by 80%. From the end of the 1990s to today, the average cholesterol value of Italians has increased significantly both in men (from 205 to 211 mg/dl) and in women (from 207 to 217 mg/dl), according to data from the Osservatorio Epidemiologico Cardiovascolare (Oec)/Health Examination Survey (Hes). The same happened for the prevalence of hypercholesterolemia, which increased from 20.8 to 34.3 percent in men and from 24 to 36.6 percent in women.

Statins are known to be used to lower cholesterol levels.

Statins are a class of drugs whose function is based on the reduction of cholesterol synthesis by the liver. Statins exert their action by inhibiting the enzyme HMG-CoA reductase (3-hydroxy-3-methylglutaryl coenzyme A reductase). This enzyme is involved in the early stages of endogenous cholesterol production and is responsible for the conversion of 3-hydroxy-3-methylglutaryl coenzyme A into mevalonic acid or mevalonate. By inhibiting this enzyme, statins reduce the cholesterol synthesis thus preventing the increase of its concentration in the bloodstream. Pharmacological therapy with statins is limited by the side effects that they can cause. These include muscle pain (myopathies), hypertransaminasemia, increased CPK. One of the hypotheses that would explain the connection between the use of statins and the appearance of muscle pain is that these drugs inhibit the formation of compounds deriving from the cholesterol synthesis process, leading to a deficit of ubiquinone (essential intracellular energy component) in mitochondria of muscle cells and thus altering normal cellular respiration.

The object of the present invention is therefore to provide a treatment for hypercholesterolemia which is effective but does not generate the side effects attributed to statins.

### SUMMARY OF THE INVENTION

The present invention is defined by the claims.

The inventors have found a combination of active ingredients which resulted to be able to effectively treat hypercholesterolemia. In particular, the inventors have identified a combination of active ingredients, some of which were already known for the treatment of hypercholesterolemia, that when combined produce a greater synergistic effect than the additive combination of known active ingredients, as will be apparent from the experimental part. Surprisingly, the composition of the invention works in the treatment of hypercholesterolemia without the use of monacolin K.

The invention therefore concerns a composition comprising a dry extract of bergamot, a dry extract of berberine (preferably from Berberis vulgaris) complexed with phospholipids, a dry extract of Ipomea batata and a dry extract of olive tree, wherein the composition does not comprise monacolin K. All active ingredients are in the form of extracts as will be detailed below.

According to the invention, said active ingredients can also be provided in combination with policosanols and vitamin E.

The invention also concerns the composition comprising a dry extract of bergamot, a dry extract of berberine (preferably from Berberis vulgaris) complexed with phospholipids, dry extract of Ipomea batata and dry extract of olive tree in oleuropein, wherein the composition does not include monacolin K, for use as a medicament.

According to a further aspect, the invention concerns the composition comprising a dry extract of bergamot, a dry extract of berberine (preferably from Berberis vulgaris) complexed with phospholipids, a dry extract of Ipomea batata; a dry extract of olive tree in oleuropein, wherein the composition does not include monacolin K, for use in the treatment of a pathology affecting the cardiovascular system selected from the group consisting of hypercholesterolemia, hypertriglyceridemia and hyperhomocysteinemia.

The invention also concerns a food supplement (further also supplement) comprising the composition of the invention and excipients, preferably for use in the treatment of a pathology affecting the cardiovascular system selected from the group consisting of hypercholesterolemia, hypertriglyceridemia and hyperhomocysteinemia, preferably in the treatment of hypercholesterolemia.

The supplement of the invention is to be used in the treatment of pathologies affecting the cardiovascular system selected from hypercholesterolemia, hypertriglyceridemia and hyperhomocysteinemia, specifically as improvement of hypercholesterolemia, wherein said improvement is selected from the group consisting of inhibition of cholesterol synthesis, reduction in the circulating LDL cholesterol concentration, reduction in the HMRG enzyme activity and triglycerides blood concentration.

It can also be used in the prevention of adverse vascular events, such as for example reduction of blood flow, thrombosis, angina pectoris, myocardial infarction, stroke.

Advantageous aspects of the composition and supplement of the invention, such as amounts, posology and dosages will be detailed in the detailed description, and the surprising synergistic effects deriving therefrom will be apparent from the subsequent experimental part.

An advantageous and surprising aspect of the invention is that the composition allows the treatment of pathologies affecting the cardiovascular system selected from hypercholesterolemia, hypertriglyceridemia and hyperhomocysteinemia without the use of monacolin K from fermented red rice, as opposed to most of the products on the market, in particular some supplements used in the treatment of heart diseases.

According to the invention, therefore, the composition of the invention does not include monacolin K.

Without being bound to any theory, the inventors believe that, in the absence of monacolin K, the composition of the invention can advantageously be used in a combined therapy with known drugs for the treatment of pathologies affecting the cardiovascular system, such as for example statins.

Therefore, in an advantageous aspect of the invention, the composition of the invention is combined with at least one statin in the treatment of a pathology affecting the cardiovascular system selected from the group consisting of hypercholesterolemia, hypertriglyceridemia and hyperhomocysteinemia.

The composition of the invention may in fact be administered alone or in combination, i.e. as a co-therapeutic agent in a fixed dose combination or in a combined therapy of separately formulated active ingredients, with at least one statin, such as for example atorvastatin, lovastatin, rosuvastatin, simvastatin.

The composition, or the supplement comprising the same, of the invention alone or advantageously in combination with at least one statin is to be used in the treatment of a pathology affecting the cardiovascular system selected from the group consisting of hypercholesterolemia, hypertriglyceridemia and hyperhomocysteinemia, preferably of hypercholesterolemia, in particular with regard to the cardiovascular risks associated therewith.

In fact, the composition of the invention, or the medicament or supplement comprising the same, has an excellent activity in inhibiting the cholesterol synthesis and the circulating LDL cholesterol concentration as well as triglycerides blood concentration and reducing the activity of the HMG-CoA reductase enzyme.

### DESCRIPTION OF THE FIGURES

Figure 1 shows the results of Example 2A;
Figure 2 shows the results of Example 2B;
Figure 3 shows the results of Example 2C;
Figure 4 shows the results of Example 2C;
Figure 5 shows the results of Example 2D;
Figure 6 shows the results of Example 2D;
Figure 7 shows the results of Example 2E;
Figure 8 shows the results of Example 2F;

### DETAILED DESCRIPTION OF THE INVENTION

The invention concerns a composition comprising a dry extract of bergamot, a dry extract of berberine complexed with phospholipids, a dry extract of Ipomea batata and a dry extract of olive tree, wherein the composition does not include monacolin K.

The composition of the invention comprises a dry extract of bergamot.

Bergamot (Citrus bergamia) is an endemic plant native to the Calabrian region in southern Italy. It has a unique profile in flavonoids and polyphenols in terms of quality/quantity. Bergamot therefore differs from other citrus fruits not only in the composition of its active ingredients, but also in their high concentration. These flavonoids compete with the action of the enzyme HMG-CoA reductase, responsible for the synthesis of endogenous cholesterol. A reduction in the lipid framework is thus obtained. In particular, the composition of the invention comprising bergamot is capable of reducing LDL levels, increasing HDL levels and reducing blood triglycerides. In the liver, endogenous cholesterol is esterified by the enzyme ACAT (Acyl-CoA cholesterol acyl-transferase) to be transported to peripheral tissues. Esterification makes the cholesterol molecule even more lipophilic and suitable for transport by plasma lipoproteins (VLDL). The composition of the invention comprising bergamot is able to inhibit the enzyme ACAT (Acyl-CoA Cholesterol Acyltransferase). Furthermore, the flavonoids present in this ingredient are able to reduce the triglycerides synthesis, through the inhibition of PAP phosphatidic acid phosphohydrolase, a key enzyme for the hepatic synthesis of triglycerides. It is known that triglycerides are made of glycerol and three fatty acids assembled together by the enzyme PAP for esterification). In order to leave the liver where they are produced towards peripheral tissues, triglycerides must be transported by VLDL lipoproteins. The composition of the invention, by inhibiting the synthesis of triglycerides, prevents their passage in the VLDLs and their transport in the blood stream, with consequent reduction in circulating triglycerides. The composition of the invention comprises a dry extract of bergamot, preferably comprising 25% of flavonoids.

The composition of the invention preferably comprises the dry extract of bergamot in an amount from about 35% to about 50% by weight with respect to the total weight of the composition, more preferably about 46% by weight with respect to the total weight of the composition.

The composition of the invention preferably comprises the dry extract of bergamot as a unitary dose per administration unit from about 100 mg to about 400 mg, more preferably of about 300 mg.

The composition of the invention comprises a dry extract of berberine, preferably from Berberis vulgaris, complexed with phospholipids, more preferably comprising 98% of berberine.

Berberine complexed with phospholipids is also referred to as liposomal berberine. Berberine is a natural inhibitor of the enzyme PCSK9 (Proprotein convertase subtilisin/kexin type 9) responsible for the reuptake and degradation of LDL lipoproteins. Berberine is an alkaloid extracted from the root of oriental plants and is characterized by a significant ability to reduce cholesterol linked to atherogenic LDL lipoproteins. The composition of the invention comprising berberine in liposomal form reduces the plasma levels of the PCSK9 enzyme, which leads to degradation of the LDL receptors. The final effect of the composition comprising berberine is therefore to increase the presence of LDL receptors on the surface of hepatocytes (and therefore the liver's ability to "capture" LDL from plasma). The decrease in the levels of the PCSK9 protein increases the presence of the receptor itself on the hepatocyte surface, and consequently increases the cellular uptake of LDLs thus reducing their plasma levels.

The inventors found that, despite the complex and multiple mechanisms of action, berberine as such had extremely low bioavailability when incorporated into the composition of the invention. For many substances, and in particular for berberine, the greatest difficulty lies not as much in penetrating the cell membrane, but in not being "expelled" from the cell again. There are, in fact, cellular extrusion mechanisms (Multi Drug Resistance, MDR) based on some specific molecules (ABC transporters) which, in the case in question, are capable of expelling large amounts of berberine from intestinal cells. Therefore, the difficulty in reaching an adequate plasma concentration of berberine, once included in the composition, is not so much due to its poor direct absorption, but to its massive elimination in the intestinal lumen by these specific transporters. And this phenomenon was particularly evident for berberine. In fact, once administered orally with the composition of the invention, berberine as such was for the most part expelled. The composition of the invention thus comprises berberine complexed with phospholipids, *i.e*. liposomal berberine. The liposomal berberine extract, thanks to the presence of the double phospholipidic membrane inside which berberine is enclosed, skips intestinal absorption and its expulsion by the enterocyte, by using the lymphatic route to reach the liver and the blood flow, and thus allowing a greater efficacy of the composition of the invention.

The composition of the invention comprises the dry extract of berberine complexed with phospholipids, preferably berberine from Berberis vulgaris.

The composition of the invention preferably comprises the dry extract of berberine complexed with phospholipids in an amount from about 20% to about 35% by weight with respect to the total weight of the composition, more preferably about 30% by weight with respect to the total weight of the composition.

The composition of the invention comprises the dry extract of berberine complexed with phospholipids as a unitary dose per administration unit from about 50 mg to about 300 mg, more preferably of about 200 mg.

The composition of the invention comprises an extract of Ipomea batata, commonly known as sweet potato.

Sweet potato is specifically the tuberous root of Ipomea batatas, a species belonging to the Convolvulaceae family, originally from Central America. Thanks to its presence, the composition of the invention provides antioxidants, vitamins, minerals, and fibers and therefore helps to protect the heart and arteries. The composition of the invention therefore comprises fibers which help to keep under control cholesterolemia and blood sugar and the high content of carbohydrates in the form of starch. The high polyphenol content of Ipomea Batata plays an important role in the antioxidant action of the composition. Polyphenols, in fact, prevent LDL cholesterol oxidation by binding to free radicals and transition metal ions to inhibit lipid peroxidase. Furthermore, the composition comprising sweet potato, thanks to the flavonoids content, can inhibit the formation of acetyl-CoA in the biosynthesis of fatty acids, a key molecule for starting the production of lipids. Furthermore, flavonoids inhibit the exogenous absorption of cholesterol, in addition to increasing biliary excretion. The composition of the invention also performs one of the most specific actions of Ipomea Batata, namely the inhibition of 3-hydroxy-3-methylglutaryl-CoA (HMG-CoA), the enzyme responsible for the synthesis of cholesterol in the liver. Inhibiting this enzyme is equivalent to significantly reducing circulating cholesterol levels. Therefore, the composition including the sweet potato flavonoids in a synergistic action with the other ingredients inhibits the esterification of cholesterol through the inhibitory action on the enzyme cholesterol acyltransferase (ACAT). This enzyme is essential to ensure that cholesterol, transported by specific lipoproteins, leaves the liver, and reaches the peripheral tissues. Also in this case, the inhibition of the enzyme ACAT results in a decrease in blood cholesterol.

The composition of the invention preferably comprises the dry extract of Ipomea batata, in an amount from about 18% to about 25% by weight with respect to the total weight of the composition, more preferably of about 15% by weight with respect to the total weight of the composition.

The composition of the invention preferably comprises the dry extract of Ipomea batata as a unitary dose per administration unit from about 50 mg to about 200, more preferably about 100 mg.

The composition of the invention also comprises a dry extract of olive tree, preferably in the form of dry extract of oleuropein, more preferably at 40% of oleuropein.

The olive extract may comprise active ingredients and/or elements from leaves, fruits, buds, oil and/or shoots.

Oleuropein and glycoside thereof are the most abundant polyphenols present in olive oil, as well as the products which give it the main organoleptic characteristics. It is present in olive leaves and fruit, together with lignans and other phenolic derivatives, such as hydroxytyrosol, and represents the most important bioactive principle of olive oil. Thanks to the presence of polyphenols in the oil, the composition of the invention has cardioprotective, neuroprotective, antiinflammatory and antioxidant properties. The antioxidant activity of oleuropein is exerted both through direct scavenger mechanisms and through the expression of antioxidant agents, and the possibility of reducing the damage induced by reactive oxygen species through the use of the composition of the invention has important repercussions especially in the cardiovascular field, such as the protection from the harmful action of oxygen free radicals applied to the oxidation of lipids. This biological event is of primary importance in the onset of atheromatous plaques caused by the accumulation of oxidized cholesterol (LDL) in the inner layers of blood vessels. Furthermore, the presence of polyphenols also deriving from the olive tree extract contained in the composition of the invention, in the forms of hydroxytyrosol and oleuropein, performs a notable inhibition action of the enzyme HMG CoA-reductase, an enzyme involved in the hepatic synthesis of cholesterol. The result of this inhibition is a significant reduction in circulating cholesterol levels. The composition of the invention comprises the dry extract of olive leaves, preferably comprising oleuropein, more preferably at 40% of oleuropein.

The composition of the invention preferably comprises the dry extract of olive leaves, preferably comprising 40% of oleuropein, in an amount from about 2% to about 6% by weight with respect to the total weight of the composition, more preferably about 3.8% by weight with respect to the total weight of the composition.

The composition of the invention preferably comprises the dry extract of olive leaves, preferably comprising 40% of oleuropein, as a unitary dose per administration unit from about 10 mg to about 50 mg, more preferably of about 25 mg.

The composition of the invention may also advantageously comprise policosanols, preferably obtained from Oryza sativa, a plant commonly known also as Asian rice, more preferably 90% of policosanols obtained from Oryza sativa.

Policosanols are natural substances that are extracted from sugar cane and some by-products of cereal processing (wheat germ, rice wax). Chemically, they are substances made of eight aliphatic alcohols, the three main ones being octacosanol, triacontanol and hexacosanol. The most important component of policosanols is octacosanol. When the composition of the invention advantageously comprises policosanols with a high percentage of octacosanol, it consistently reduces cholesterol not by acting directly on the enzyme HMG-CoA reductase and competitively or non-competitively inhibiting its activity, but by inhibiting the gene which codifies the enzyme expression. This results in the reduction of the HMG enzyme amount, which consequently will produce less cholesterol. Furthermore, thanks to the presence of policosanols, the composition of the invention helps to reduce lipid oxidation by limiting the LDL lipoprotein sensitivity to oxidative damage. The active ingredients of policosanols, thanks to their antiplatelet action, prevent the possible formation of thrombi, the triggering cause of heart attacks, ischemia, and arterial insufficiency.

The composition of the invention preferably comprises polycosanols in an amount from about 1% to about 3% by weight with respect to the total weight of the composition, more preferably of about 1.7% by weight with respect to the total weight of the composition.

The composition of the invention preferably comprises polycosanols as a unitary dose per administration unit from about 5 mg to about 20 mg, more preferably of about 11 mg.

The composition of the invention may also include vitamin E.

Biological membranes act as a partition between cells and their environment. Under oxidative stress, unsaturated lipids, present in cell membranes, can be exposed to attacks by free radicals, *i.e*. oxidation. Oxidation transforms some of the membrane lipids and alters the properties of the bilayer. The alterations occur as the oxidized chains are energetically unfavored to remain inside the lipid bilayer, and therefore they are reversed; as a result, there is a reversal of the lipid chain with large changes in the bilayer properties, such as an increase in the area for the lipids, thinning of the bilayers, and an increase in water permeability. The composition of the invention comprising vitamin E (alpha-tocopherol) has properties of effective antioxidant, which protects membranes from oxidation initiated by free radicals. Membranes are normally made of saturated and unsaturated lipids and are permeable to water and small molecules; unsaturated lipids play an important role in membrane permeability, disrupting the packaging of saturated lipids. However, they are easily susceptible to peroxidation which, if extreme, can lead to an uncontrollable transport of molecules across the membrane. The composition of the invention comprising vitamin E molecules prefers to interact with oxidized lipids, compared to a non-oxidized system, thus remaining inside the oxidized bilayer; forming hydrogen bonds with these lipids tails, which are able to stabilize the chains and reduce the conformational changes phenomena. Free radicals play an important role in the damage of biological membranes and oxidized lipids are the main product causing bilayer deformation and cardiovascular problems. The composition can therefore inhibit pores formation in oxidized lipid bilayers, and by controlling the concentration thereof, can increase the stability of biological membranes.

The composition of the invention preferably comprises vitamin E in an amount from about 1% to about 4% by weight with respect to the total weight of the composition, more preferably of about 2.3% by weight with respect to the total weight of the composition.

The composition of the invention preferably comprises vitamin E as a unitary dose per administration unit from about 10 mg to about 30 mg, more preferably of about 15 mg.

The composition of the invention therefore resulted to be a selected and synergistic combination of active ingredients capable of treating hypercholesterolemia.

According to a primary aspect of the invention, the invention concerns a composition comprising the composition of the invention for use as a medicament. According to a further aspect, the invention concerns the composition described above for use in the treatment of a pathology affecting the cardiovascular system selected from the group consisting of hypercholesterolemia, hypertriglyceridemia and hyperhomocysteinemia, preferably in the treatment of hypercholesterolemia. The invention also concerns a supplement comprising the composition of the invention and excipients.

The supplement of the invention is to be used in the treatment of pathologies affecting the cardiovascular system selected from hypercholesterolemia, hypertriglyceridemia and hyperhomocysteinemia, specifically as improvement of hypercholesterolemia, wherein said improvement is selected from the group consisting of inhibition of cholesterol synthesis, reduction in the circulating LDL cholesterol concentration, reduction in the HMRG enzyme activity and triglycerides blood concentration.

It may also be used in the prevention of adverse vascular events, such as for example reduction of blood flow, thrombosis, angina pectoris, myocardial infarction, stroke.

An advantageous and surprising aspect of the invention is that the composition allows the treatment of pathologies affecting the cardiovascular system selected from hypercholesterolemia, hypertriglyceridemia and hyperhomocysteinemia without the use of monacolin K from fermented red rice in contrast with most products on the market, in particular some supplements used in the treatment of heart diseases.

According to the invention, therefore, the composition of the invention does not comprise monacolin K.

The composition and supplement for use in the treatment of pathologies affecting the cardiovascular system selected from hypercholesterolemia, hypertriglyceridemia and hyperhomocysteinemia, may be administered preferably by oral administration.

The compositions may therefore be in liquid, solid or semi-solid form and may be in the form of tablets, pearls, drops, sticks, syrups, sachets.

The compositions of the invention may be administered orally in the form of tablets, capsules, granules, effervescent granules, syrups or the like.

They may also be soluble in water to form inhalable solutions.

The solid form compositions may include one or more additives such as starches, sugars, cellulose, diluents, granulating agents, lubricants, binders, disintegrating agents, stabilizers, emulsifiers, texturizers, plasticizers, wetting agents, thickeners, coatings. All these excipients make it possible to obtain the supplement of the invention in the desired pharmaceutical formulation.

The capsules can be coated with a gastro-resistant film, or they can be soft gel capsules.

Flavoring agents, preservatives, coloring agents or the like may also be present. The active ingredients contained in the compositions object of the present invention may be combined or mixed as known active ingredients and/or excipients according to pharmaceutical and food or nutritional industry techniques, and prepared by methods known in the pharmaceutical or food industry.

The compositions object of the invention are also used as adjuvants in the treatment therapies of pathologies affecting the cardiovascular system selected from hypercholesterolemia, hypertriglyceridemia and hyperhomocysteinemia.

Without being tied to any theory, the inventors believe that, in the absence of monacolin K, the composition of the invention may be advantageously used in a combined therapy with known drugs for the treatment of pathologies affecting the cardiovascular system, such as for example statins.

Therefore, in an advantageous aspect of the invention, the composition of the invention is combined with at least one statin in the treatment of a pathology affecting the cardiovascular system selected from the group consisting of hypercholesterolemia, hypertriglyceridemia and hyperhomocysteinemia.

The composition of the invention may, in fact, be administered alone or in combination, *i.e*. as a co-therapeutic agent in a fixed dose combination or in a combined therapy of separately formulated active ingredients, with at least one statin, such as for example atorvastatin, lovastatin, rosuvastatin, simvastatin.

The composition, or the medicament or supplement comprising the same, of the invention, alone or advantageously in combination with at least one statin, resulted to be effective in the treatment of a pathology affecting the cardiovascular system selected from the group consisting of hypercholesterolemia, hypertriglyceridemia and hyperhomocysteinemia, preferably in the treatment of hypercholesterolemia, in particular in relation to the cardiovascular risks associated therewith.

The composition, or the medicament or supplement comprising the same, of the invention, alone or advantageously in combination with at least one statin, may also be used in the prevention of adverse vascular events, such as for example reduction of blood flow, thrombosis, angina pectoris, myocardial infarction, stroke. In fact, the composition of the invention, or the medicament or supplement comprising the same, has an excellent ability to deprive cholesterol from the blood flow, positively influencing the LDL lipoprotein receptor, and an excellent ability to significantly reduce the activity of the enzyme HMRG, in the case of multiple administrations of the composition, reaching reduction values equal to about 60% and about 85%, when administered 2 or 3 times, respectively.

As will be apparent from the experimental part, the composition of the invention containing liposomal berberine extract was found to be about 20% more effective than the one containing the dry extract, as indicated above, probably due to a greater cellular penetration of the berberine liposomal form when in contact with the cell membrane, showing an inhibition capacity of about 85%, with respect to the control.

In particular, the composition of the invention is therefore useful as a dietary and nutritional supplement, as a medicament, for the prevention and treatment of pathologies affecting the cardiovascular system selected from hypercholesterolemia, hypertriglyceridemia and hyperhomocysteinemia.

The compositions object of the invention are therefore pharmaceutical preparations or food supplements which can be introduced into the diet regimen of an individual suffering from pathologies affecting the cardiovascular system selected from hypercholesterolemia, hypertriglyceridemia and hyperhomocysteinemia.

The composition object of the present invention is therefore a valid therapeutic tool for the management of hypercholesterolemia, hypertriglyceridemia and hyperhomocysteinemia.

The advantageous and preferable effect is obtained when the dose of the composition of the present invention, as regards to the single administration unit, respects the following characteristics:
i) Bergamot dry extract is present in a dose from 100 to 400 mg, preferably of 300 mg.
ii) Berberis vulgaris dry extract complexed with phospholipids is present in a dose from 50 to 300 mg, preferably of 200 mg
iii) Ipomea Batata dry extract is present in a dose of from 50 to 200 mg, preferably of 100 mg.
iv) Olive dry extract in oleuropein is present in a dose from 10 to 50 mg, preferably of 25 mg.
v) Policosanols are present in a dose from 5 to 20 mg, preferably of 11.1 mg.
vi) Vitamin E is present in a dose from 10 to 30 mg, preferably of 15 mg.

The synergism generated by the above combination, in the absence of monacolin k, has shown its efficacy in enhancing the effects of its components which was greater than the sum of the known effects of the individual active ingredients.

### EXPERIMENTAL PART

### Example 1: Preparation of the composition of the invention

The composition of the invention was prepared using the geometric dilution method so as to obtain a homogeneous mixing of the powders in the mortar. The composition was prepared with the following ingredients in the indicated amounts:
- The dry extract of bergamot was present in an amount of 300 mg
- The dry extract of Berberis vulgaris complexed with phospholipids at 98% berberine was present in an amount of 200 mg
- The dry extract of Ipomea Batata was present in an amount of 100 mg
- The dry extract of olive leaves (40% oleuropein) was present in an amount of 15 mg.

The above composition could also include:
- Policosanols (90% from Oryza sativa) are present in an amount of 11 mg and
- Vitamin E is present in an amount of 15 mg.

The compositions of the invention could be formed by combining the above ingredients in the indicated amounts, or amounts falling within the ranges indicated above.

### Example 2: Evaluation of the composition of the invention.

### Example 2A

### PCSK9 INHIBITION IN HEPG2 LIVER CELLS

Immortalized HepG2 human hepatocellular carcinoma cells were cultured and then treated with berberine extract, and the composition of Example 1. After treatment, the cells were lysed and the mRNA (messenger RNA) levels for PCSK9 expression were evaluated by qRT-PCR using human-specific PCR primers. For each sample, the measurements were carried out at least in triplicate and the results were reported as the average of the single values.

The efficacy of the invention composition was tested against HepG2 immortalized human hepatocarcinoma cells. It is appropriate to underline the importance of this type of analysis and how it can demonstrate the hypolipidemic action of the composition of the invention. The inhibition of the messenger RNA (mRNA) encoding the translation of the protein PCSK9 plays a key role in the mechanisms of action aimed at maximizing the hepatic reuptake of LDL cholesterol. A reduced PCSK9 translation increases the number of hepatic LDL receptors (LDLRs) which perform their role of depriving cholesterol from the bloodstream, reducing the concentration thereof.

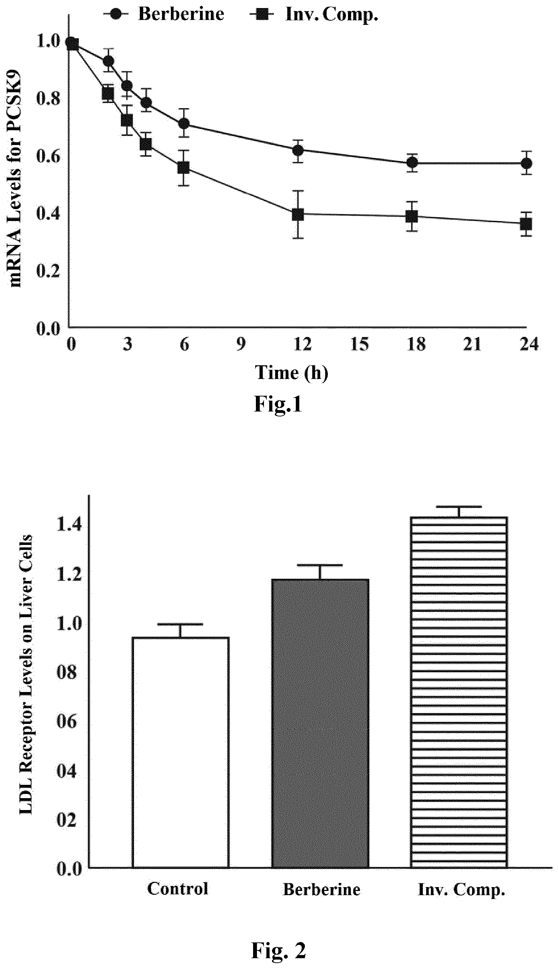

As it is possible to see in Figure 1, the mRNA levels related to PCSK9 expression of HepG2 cells are influenced by treatment with 40 µM of berberine, showing a reduction of about 25% after 6 hours and of 40% after 24 hours, compared to the control. The composition of Example 1 showed a significantly more marked activity than berberine alone, when administered in an amount of 40 µM equivalent berberine. In fact, a reduction of more than 40% was recorded after 6 hours from the treatment, while at 24 hours inhibition levels equal to about 60% were detected, due to a synergistic effect also of the other components present in the mixture, capable of influencing the PCSK9-LDLR metabolic pathway.

### Example 2B.

### LDL RECEPTOR EXPRESSION IN HEPG2 LIVER CELLS

Immortalized HepG2 human hepatocellular carcinoma cells were treated with berberine, the composition of Example 1 and a control (DMSO dimethyl sulfoxide). After treatment, the cells were lysed, and the cell lysates were used for immunoblotting using anti-LDLR antibodies. The measurements were carried out in triplicate for each sample and the results were reported as the average of the single values. HepG2 cells were subjected to studies for the determination of the LDLR protein abundance. Since the PCSK9 levels are strictly correlated to those of LDLR, the graph in Figure 2 shows how the composition of the invention is able to influence LDLR levels in liver cells through up-regulation in a more significant way compared to the extract of berberine alone. Also in this case, therefore, the Invention mixture was able to positively influence the LDL receptor in the liver in a more marked way than berberine alone, when dosed at the same amount.

### Example 2C.

### EFFECT OF SELECTED COMPOUNDS ON HUMAN HMG ACTIVITY

First-line human liver cells (HUCPI) were cultured and treated with some compositions, and with atorvastatin as a control, to evaluate the inhibition capacity of the HMG CoA-reductase enzyme. The enzymatic activity of HMG-CoA reductase was evaluated by measuring the dependent oxidation of NAPDH. For each sample, the measurements were carried out at least in triplicate, and the results were reported as the average of the single values.

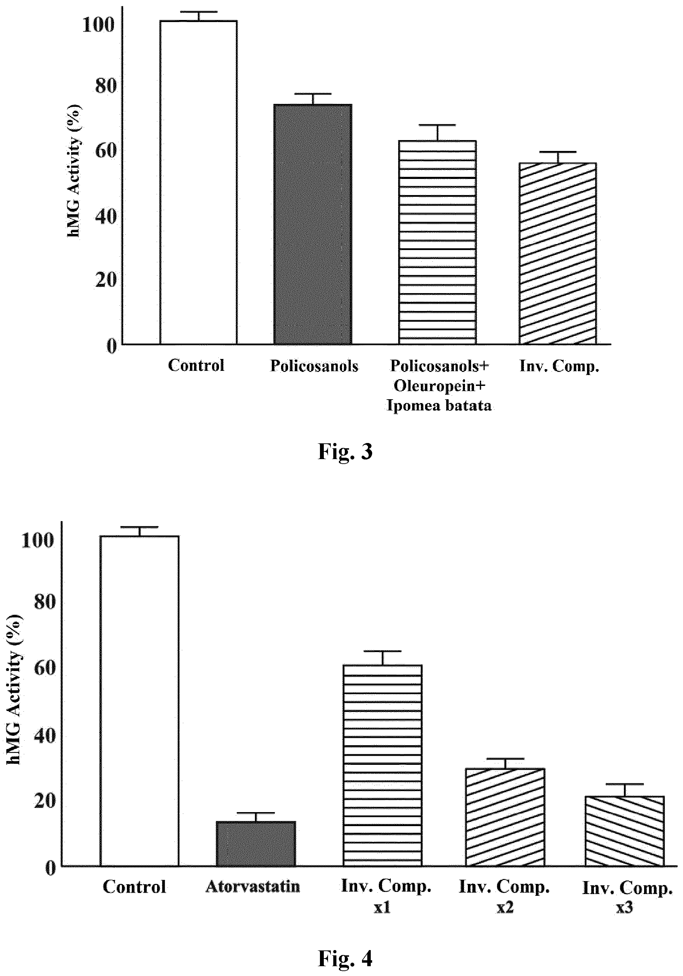

The HUCPI cells were treated with the composition of Example 1 and with solutions of single extracts of Policosanols, and a mixture containing Policosanols, Oleuropein and Ipomea batata, in amounts equivalent to the ingredients of the composition of Example 1. After treatment, the cells were lysed and the catalytic fragment of the protein HMGR was cloned, overexpressed, and purified. As can be seen from Figure 3, the different extracts are able to significantly reduce HMGR activity at the tested concentrations. Furthermore, the composition of the invention showed an ability to reduce HMGR activity by about 35% compared to the control, by 10% compared to the mixture consisting of Policosanols, Oleuropein and Ipomea batata, and by about 25% more effectively than the solution containing Policosanols. Figure 4 shows a comparison of human HMG-CoA reductase activity in liver cells. As can be seen, the treatment with atorvastatin administered once in the 24-hour period of the experiment significantly reduced the activity of HMRG. At the same time the composition of the invention was shown to be able to significantly reduce HMRG activity, reaching, in the case of multiple administrations (x1, x2 and x3), values equal to about 60% and about 85%, when administered 2 or 3 times, respectively.

### Example 2D.

### ACYLCOA-CHOLESTEROL ACYLTRANSFERASE (ACAT) ENZIME LEVELS

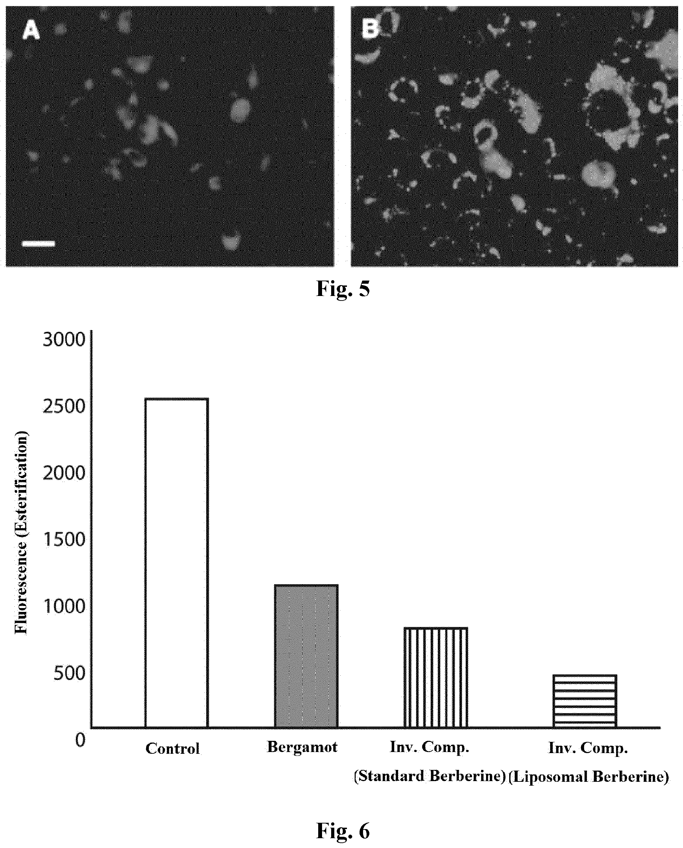

Immortalized human HepG2 hepatocellular carcinoma cells were transfected (addition of genetic material) with genes and human ACAT via plasmids. ACAT plays the role of esterification of hepatic cholesterol. The cells were treated with fluorescence so that the pre-NBD free cholesterol was esterified and visible. After reading the dishes containing the incubated and washed cells. The internal fluorescence was calculated by subtracting baseline value from total emitted fluorescence. For each sample, the measurements were carried out at least in triplicate, and the results were reported as the average of the single values. Figure 5 shows native and transfected HepG2 cells, demonstrating the increase in fluorescence when NBD cholesterol is esterified. The fluorescence variation due to the esterification was used to determine the activity of the enzyme ACAT before and after treatment with the different compounds and the composition of Example 1. Figure 6 shows the fluorescence levels found after treatment of the cells with Bergamot extract or with the composition of the present invention. As can be seen, ACAT activity levels are reduced by more than 50% after treatment with a Bergamot solution. This inhibition is further significantly intensified when the cells are treated with the composition of the invention, thanks to the synergistic effect with the other ingredients. Furthermore, a significant difference was noted when the treatment involved the use of the composition of the invention containing a Berberine extract in liposomal form. In fact, the composition of the invention containing Berberine extract in liposomal form was found to be about 20% more effective than the one containing the extract in dry form, probably due to a greater cellular penetration of the liposomal form when in contact with the cell membrane, showing an inhibition capacity of about 85%, compared to the control.

### Example 2E.

### Measurement of ROS levels in epithelium cells of murine penile muscle

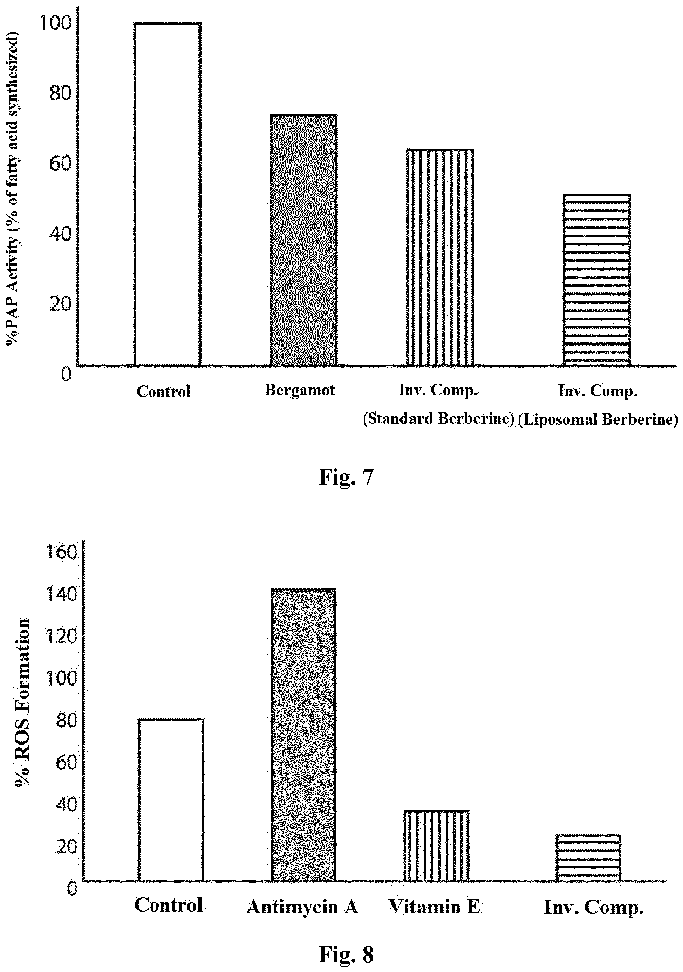

This experiment was carried out to test the phosphatidic acid phosphatase (PAP) activity of human hepatocyte cells. The enzyme PAP has the task of assembling triglycerides starting from glycerol + 3 fatty acids. The cells were treated with the bergamot extract, the control (DMSO dimethyl sulfoxide), including the composition of Example 1, and subsequently phosphatidic acid was used as substrate of the enzyme added to the culture medium. The diacylglycerol (fatty acid) generated by the reaction was measured by LC-MS/MS performed on a mass spectrometer. The various components of the organic phase were separated using a chromatographic column to evaluate the PAP enzyme activity. The PAP enzyme activity was tested in HepG2 liver cells as a function of the amount of total lipid extracts from the cultures treated with the bergamot extract or with the entire composition of the invention, as quantified by HPLC MS/MS method. As can be seen from Figure 7, the greatest inhibitory activity of phosphatidic acid phosphatase activity was shown by the composition of the invention containing the Berberine extract in liposomal form. The inhibitory activity proved to be approximately 50% higher than in control conditions, compared to Bergamot, in equal amounts, which demonstrated an inhibitory capacity of the enzyme PAP of 32%.

### Example 2F.

### DETERMINATION OF REACTIVE OXYGEN SPECIES FORMATION

To evaluate the total cellular amount of ROS (reactive oxygen species) after stimulation with antimycin A, human liver cells were fluorescence treated. The fluorescence intensity was measured using a microplate fluorometer. To evaluate the effect of the composition object of the present invention as an antioxidant agent, the ability of the same and other single biomolecules to act on the total ROS cellular amount was evaluated using HUCPI cells (cryopreserved human hepatocytes). Once ready for the test, the cells were added with solutions at a concentration of vitamin E, Bergamot extract and with the composition of Example 1. As can be seen from Figure 8, a still marked ability to inhibit cellular ROS production compared to the control was demonstrated by the composition object of the present invention, when compared to both vitamin E and Bergamot, thanks to the good flavonoid content.

## Claims

1. A composition comprising a dry extract of bergamot, a dry extract of berberine complexed with phospholipids, a dry extract of Ipomea batata and a dry extract of olive tree, wherein the composition does not comprise monacolin K.

2. The composition according to claim 1, wherein the dry extract of bergamot, preferably comprises 25% of flavonoids.

3. The composition according to claim 1 or claim 2, wherein the dry extract of bergamot is in an amount from about 35% to about 50% by weight with respect to the total weight of the composition, preferably about 46% by weight with respect to the total weight of the composition.

4. The composition according to anyone of claims 1 to 3, wherein the composition comprises the dry extract of bergamot as a unitary dose per administration unit from about 100 mg to about 400 mg, preferably of about 300 mg.

5. The composition according to anyone of claims 1 to 4, wherein the composition comprises the dry extract of berberine complexed with phospholipids in an amount from about 20% to about 35% by weight with respect to the total weight of the composition, preferably of about 30% by weight with respect to the total weight of the composition.

6. The composition according to anyone of claims 1 to 4, wherein the composition comprises the dry extract of berberine complexed with phospholipids as a unitary dose per administration unit from about 50 mg to about 300 mg, preferably of about 200 mg.

7. The composition according to anyone of claims 1 to 6, wherein the composition comprises the dry extract of Ipomea batata, in an amount from about 18% to about 25% by weight with respect to the total weight of the composition, preferably of about 15% by weight with respect to the total weight of the composition.

8. The composition according to anyone of claims 1 to 6, wherein the composition comprises the dry extract of Ipomea batata as a unitary dose per administration unit from about 50 mg to about 200 mg, preferably of about 100 mg.

9. The composition according to anyone of claims 1 to 8, wherein the composition comprises the dry extract of olive tree in the form of dry extract of olive leaves, preferably comprising oleuropein, more preferably a 40% amount of oleuropein.

10. The composition according to any one of claims 1 to 9, wherein the composition comprises the dry extract of olive tree, as a unitary dose per administration unit, in an amount from about 10 mg to about 50 mg, preferably of about 25 mg.

11. A composition according to anyone of claims 1 to 10 for use as a medicament.

12. A composition according to anyone of claims 1 to 10 for use in the prevention or in the treatment of a pathology affecting the cardiovascular system selected from the group consisting of hypercholesterolemia, hypertriglyceridemia and hyperhomocysteinemia, preferably in the treatment of hypercholesterolemia.

13. A food supplement comprising the composition according to anyone of claims 1 to 10 and excipients.

14. A food supplement comprising the composition according to any one of claims 1 to 10, for use in the prevention or in the treatment of a pathology affecting the cardiovascular system selected from the group consisting of hypercholesterolemia, hypertriglyceridemia and hyperhomocysteinemia, preferably in the treatment of hypercholesterolemia, specifically as an improvement in hypercholesterolemia, wherein said improvement is selected from the group consisting of inhibition of cholesterol synthesis, reduction in the circulating LDL cholesterol concentration, reduction in the HMRG enzyme activity and in triglycerides blood concentration.

15. A composition according to anyone of claims 1 to 10 alone or in combination, *i.e.* as a co-therapeutic agent in a fixed dose combination or in a combined therapy of separately formulated active ingredients, with a statin for use in the prevention or in the treatment of pathologies affecting the cardiovascular system selected from hypercholesterolemia, hypertriglyceridemia and hyperhomocysteinemia.

## Patentansprüche

1. Zusammensetzung, die einen Trockenextrakt aus Bergamotte, einen Trockenextrakt aus mit Phospholipiden komplexiertem Berberin, einen Trockenextrakt aus Ipomea batata und einen Trockenextrakt aus Olivenbaum umfasst, wobei die Zusammensetzung kein Monacolin K umfasst.

2. Zusammensetzung nach Anspruch 1, wobei der Trockenextrakt aus Bergamotte vorzugsweise 25% Flavonoide umfasst.

3. Zusammensetzung nach Anspruch 1 oder Anspruch 2, wobei der Trockenextrakt aus Bergamotte in einer Menge von etwa 35 Gew.-% bis etwa 50 Gew.-%, bezogen auf das Gesamtgewicht der Zusammensetzung, vorzugsweise etwa 46 Gew.-%, bezogen auf das Gesamtgewicht der Zusammensetzung, vorliegt.

4. Zusammensetzung nach einem der Ansprüche 1 bis 3, wobei die Zusammensetzung den Trockenextrakt aus Bergamotte als Einheitsdosis pro Verabreichungseinheit von etwa 100 mg bis etwa 400 mg, vorzugsweise etwa 300 mg, umfasst.

5. Zusammensetzung nach einem der Ansprüche 1 bis 4, wobei die Zusammensetzung den Trockenextrakt aus mit Phospholipiden komplexiertem Berberin in einer Menge von etwa 20 Gew.-% bis etwa 35 Gew.-%, bezogen auf das Gesamtgewicht der Zusammensetzung, vorzugsweise etwa 30 Gew.-%, bezogen auf das Gesamtgewicht der Zusammensetzung, umfasst.

6. Zusammensetzung nach einem der Ansprüche 1 bis 4, wobei die Zusammensetzung den Trockenextrakt aus mit Phospholipiden komplexiertem Berberin als Einheitsdosis pro Verabreichungseinheit von etwa 50 mg bis etwa 300 mg, vorzugsweise etwa 200 mg, umfasst.

7. Zusammensetzung nach einem der Ansprüche 1 bis 6, wobei die Zusammensetzung den Trockenextrakt aus Ipomea batata in einer Menge von etwa 18 Gew.-% bis etwa 25 Gew.-%, bezogen auf das Gesamtgewicht der Zusammensetzung, vorzugsweise etwa 15 Gew.-%, bezogen auf das Gesamtgewicht der Zusammensetzung, umfasst.

8. Zusammensetzung nach einem der Ansprüche 1 bis 6, wobei die Zusammensetzung den Trockenextrakt aus Ipomea batata als Einheitsdosis pro Verabreichungseinheit von etwa 50 mg bis etwa 200 mg, vorzugsweise etwa 100 mg, umfasst.

9. Zusammensetzung nach einem der Ansprüche 1 bis 8, wobei die Zusammensetzung den Trockenextrakt aus Olivenbaum in Form eines Trockenextrakts aus Olivenblättern umfasst,
der vorzugsweise Oleuropein, insbesondere eine Menge von 40% Oleuropein, umfasst.

10. Zusammensetzung nach einem der Ansprüche 1 bis 9, wobei die Zusammensetzung den Trockenextrakt aus Olivenbaum als Einheitsdosis pro Verabreichungseinheit in einer Menge von etwa 10 mg bis etwa 50 mg, vorzugsweise etwa 25 mg, umfasst.

11. Zusammensetzung nach einem der Ansprüche 1 bis 10 zur Verwendung als Medikament.

12. Zusammensetzung nach einem der Ansprüche 1 bis 10 zur Verwendung bei der Vorbeugung oder Behandlung einer das Herz-Kreislauf-System betreffenden Pathologie, ausgewählt aus der Gruppe, bestehend aus Hypercholesterinämie, Hypertriglyceridämie und Hyperhomocysteinämie, vorzugsweise bei der Behandlung von Hypercholesterinämie.

13. Nahrungsergänzungsmittel, das die Zusammensetzung nach einem der Ansprüche 1 bis 10 und Hilfsstoffe umfasst.

14. Nahrungsergänzungsmittel, das die Zusammensetzung nach einem der Ansprüche 1 bis 10 umfasst, zur Verwendung bei der Vorbeugung oder Behandlung einer das Herz-Kreislauf-System betreffenden Pathologie, ausgewählt aus der Gruppe, bestehend aus Hypercholesterinämie, Hypertriglyceridämie und Hyperhomocysteinämie, vorzugsweise bei der Behandlung von Hypercholesterinämie, insbesondere zur Verbesserung der Hypercholesterinämie, wobei die Verbesserung aus der Gruppe, bestehend aus Hemmung der Cholesterinsynthese, Verringerung der Konzentration von zirkulierendem LDL-Cholesterin, Verringerung der HMRG-Enzymaktivität und der Triglyceridkonzentration im Blut ausgewählt ist.

15. Zusammensetzung nach einem der Ansprüche 1 bis 10 allein oder in Kombination, d. h. als Co-Therapeutikum in einer Fixdosiskombination oder in einer Kombinationstherapie aus separat formulierten Wirkstoffen, mit einem Statin zur Verwendung bei der Vorbeugung oder Behandlung von das Herz-Kreislauf-System betreffenden Pathologien, ausgewählt aus Hypercholesterinämie, Hypertriglyceridämie und Hyperhomocysteinämie.

## Revendications

1. Composition comprenant un extrait sec de bergamote, un extrait sec de berbérine complexé avec des phospholipides, un extrait sec d'Ipomea batata et un extrait sec d'olivier, dans laquelle la composition ne comprend pas de monacoline K.

2. Composition selon la revendication 1, dans laquelle l'extrait sec de bergamote comprend de préférence 25 % de flavonoïdes.

3. Composition selon la revendication 1 ou la revendication 2, dans laquelle l'extrait sec de bergamote est en une quantité d'environ 35 % à environ 50 % en poids par rapport au poids total de la composition, de préférence environ 46 % en poids par rapport au poids total de la composition.

4. Composition selon l'une quelconque des revendications 1 à 3, dans laquelle la composition comprend l'extrait sec de bergamote en tant que dose unitaire par unité d'administration d'environ 100 mg à environ 400 mg, de préférence environ 300 mg.

5. Composition selon l'une quelconque des revendications 1 à 4, dans laquelle la composition comprend l'extrait sec de berbérine complexé avec des phospholipides en une quantité d'environ 20 % à environ 35 % en poids par rapport au poids total de la composition, de préférence environ 30 % en poids par rapport au poids total de la composition.

6. Composition selon l'une quelconque des revendications 1 à 4, dans laquelle la composition comprend l'extrait sec de berbérine complexé avec des phospholipides en tant que dose unitaire par unité d'administration d'environ 50 mg à environ 300 mg, de préférence environ 200 mg.

7. Composition selon l'une quelconque des revendications 1 à 6, dans laquelle la composition comprend l'extrait sec d'Ipomea batata, en une quantité d'environ 18 % à environ 25 % en poids par rapport au poids total de la composition, de préférence environ 15 % en poids par rapport au poids total de la composition.

8. Composition selon l'une quelconque des revendications 1 à 6, dans laquelle la composition comprend l'extrait sec d'Ipomea batata en tant que dose unitaire par unité d'administration d'environ 50 mg à environ 200 mg, de préférence environ 100 mg.

9. Composition selon l'une quelconque des revendications 1 à 8, dans laquelle la composition comprend l'extrait sec d'olivier sous forme d'extrait sec de feuilles d'olivier,
comprenant de préférence de l'oleuropéine, plus préférablement une quantité de 40 % d'oleuropéine.

10. Composition selon l'une quelconque des revendications 1 à 9, dans laquelle la composition comprend l'extrait sec d'olivier, en dose unitaire par unité d'administration, en une quantité d'environ 10 mg à environ 50 mg, de préférence environ 25 mg.

11. Composition selon l'une quelconque des revendications 1 à 10, destinée à être utilisée comme médicament.

12. Composition selon l'une quelconque des revendications 1 à 10, destinée à être utilisée dans la prévention ou dans le traitement d'une pathologie affectant le système cardiovasculaire choisie dans le groupe constitué par l'hypercholestérolémie, l'hypertriglycéridémie et l'hyperhomocystéinémie, de préférence dans le traitement de l'hypercholestérolémie.

13. Complément alimentaire comprenant la composition selon l'une quelconque des revendications 1 à 10, et des excipients.

14. Complément alimentaire comprenant la composition selon l'une quelconque des revendications 1 à 10, destiné à être utilisé dans la prévention ou dans le traitement d'une pathologie affectant le système cardiovasculaire choisie dans le groupe constitué par l'hypercholestérolémie, l'hypertriglycéridémie et l'hyperhomocystéinémie, de préférence dans le traitement de l'hypercholestérolémie, en particulier comme amélioration de l'hypercholestérolémie, dans lequel ladite amélioration est choisie dans le groupe constitué par l'inhibition de la synthèse du cholestérol, la réduction de la concentration de cholestérol LDL circulant, la réduction de l'activité de l'enzyme HMRG et de la concentration de triglycérides dans le sang.

15. Composition selon l'une quelconque des revendications 1 à 10, seule ou en combinaison, c'est-à-dire en tant qu'agent co-thérapeutique dans une combinaison à dose fixe ou dans une thérapie combinée d'ingrédients actifs formulés séparément, avec une statine destinée à être utilisée dans la prévention ou dans le traitement de pathologies affectant le système cardiovasculaire choisies parmi l'hypercholestérolémie, l'hypertriglycéridémie et l'hyperhomocystéinémie.
